# EUROPEAN PATENT APPLICATION

(11) **EP 4 102 517 A1**
(43) Date of publication of application: **14.12.2022**
(21) Application number: 21179099.3
(22) Date of filing: 11.06.2021
(51) Int. Cl.: G16H 50/30

(54) **RISK DETERMINATION FOR A CT-EXAMINATION**

(71) Applicant: Siemens Healthcare GmbH, 91052 Erlangen (DE)
(72) Inventor: Gutjahr, Ralf, 90459 Nürnberg (DE); Kuo, Yu Wen, Shanghai 201204 (CN); Zhao, Yufan, 91052 Erlangen (DE)

(57) **Abstract**

The invention describes a risk indicator device (6) designed for risk determination for a CT-examination, comprising:
- a data interface (10) designed for receiving a dataset (D) of a patient (P), the dataset (D) comprising patient demographic data, data about previous treatments and data about medication of the patient (P),
- a risk indicator unit (11) designed to evaluate a risk-probability (R) for the CT-examination based on the patient demographic data, the data about the previous treatments and the data about the medication of the patient (P),
- an output unit (12) designed to output information (O) and/or control data (C) based on the evaluated risk-probability (R).

The invention further describes a respective method, a related risk prediction model, a patient scheduler and a CT-system.

## Description

The invention relates to a risk indicator device and a method for risk determination for a CT-examination, especially for risk indication of a CT-examination. Thus, the invention is advantageous for predicting (and displaying) risks for a patient going through a CT-examination.

Computed tomography (CT) is a medical imaging technique used in radiology to get detailed 2D or 3D images of the body for diagnostic purposes. CT-examinations are an important backbone of modern medicine.

CT scanners use a rotating X-ray tube and a number of detectors placed opposite of the tube to measure attenuations of the X-rays when passing a patient or an object inside the scanner. Typically, there are made multiple X-ray measurements taken from different angles in order to reconstruct 3D images of a region of interest.

Although CT-examinations are relatively safe procedures, there are nevertheless some risks, since there is used ionizing radiation (X-rays) for a CT-scan and sometimes contrast agents are applied to a patient to visualize certain regions. These risks are different for different patients. For example, children are more sensitive to radiation exposure than adults another example is a person who are allergic to contrast agents resulting in a (sometimes life-threatening) allergic reaction during or after examination.

At a typical contrast enhanced examination ("Enhanced CT"), a contrast agent is injected into a vein, a CT scan is performed, and the resulting data is processed through a computer to obtain an image. During this examination, the following main risks may occur. First, the contrast agent can cause allergies to the human body and may cause transient damage to the kidney. Second, CT scans are radiographic scans, which can also cause certain damage to the body. In addition, when the contrast agent is injected, a large-dose bolus is required for a short time. Some patients with heart insufficiency may experience aggravated heart failure.

Concerning these risks, patients may be informed previous the examination. However, analysis shows that often patients are not given information about the risks, benefits, and radiation dose for a CT scan (see e.g. Lee, C.I. et al. "Diagnostic CT Scans: Assessment of Patient, Physician, and Radiologist Awareness of Radiation Dose and Possible Risks", RSNA, Radiology, Vol. 231, No. 2, 2004). It seems that patients, physicians, and radiologists alike are unable to provide accurate estimates of CT risks regardless of their experience level.

Currently, patients have to tell a doctor about any sensitivities to medications or any kidney problems and other factors that may increase the patient's risk. There are many factors that need to be taken into consideration. But mainly it depends on the doctor's subjective judgement to rate the risk of a patient of a CT scan. There are various questionnaires in different format and many times in paper format to document the evaluation. But until now there is no systematic and automatic way of evaluating patient's risk and indicate the risks directly on a CT scan workflow.
Current patient care functionalities focus on automatic dose modulation and contrast medium administration during the scan. But there are no known functions that achieve to predict patient risk with automatic integration of data sources and machine learning models.
It is the object of the present invention to improve the known devices and methods to facilitate a better risk determination for a CT-examination.

This object is achieved by a risk indicator device according to claim 1, a method according to claim 7 risk prediction model according to claim 11, a patient scheduler according to claim 12 and a CT-system according to claim 13.

The invention relates to a risk indicator device designed for risk determination for a CT-examination, comprising:
- a data interface designed for receiving a dataset of a patient, the dataset comprising patient demographic data, data about previous treatments and data about medication of the patient,
- a risk indicator unit designed to evaluate a risk-probability for the CT-examination based on the dataset of the patient, in particular based on the patient demographic data, the data about the previous treatments and the data about the medication of the patient,
- an output unit designed to output output information and/or control data based on the evaluated risk-probability.

The risk indicator device could be an independent device. However, it could also be present as a function in a higher-ranking device. For example, the risk indicator device could be a function of a patient scheduler or a CT control device. In this case, the risk indicator device could also be designated "risk indicator function".
The risk indicator device is preferably designed for determining contrast allergic risks and overdose risks. For both determinations, patient demographic data, data about previous treatments and data about medication of the patient are needed. For effectively determining contrast allergic risks, it is very advantageous to also have data of the group allergic dispositions (of the patient), recent lab results (of examinations of the patient), previous exams and/or diagnosis (of the patient).

Preferred patient demographic data comprises data concerning gender, age, weight, race, or pregnancy of the patient.

Preferred data of allergic dispositions comprises data concerning the existence of asthma, iodine-allergy, or beta blocker-allergy of the patient.

Preferred recent lab results comprises data concerning lab results of the patient, especially the existence of creatinine in the patient.

Preferred previous exam and diagnosis data comprises data concerning diseases of the patient, especially hypertension, heart failure, myeloma, or diabetes.

Preferred data about previous treatments comprises data concerning interventions (executed or necessary) or the insertion of objects into the patient, especially central venous catheter, kidney surgery, chemotherapy, or dialysis.

Preferred data about medication comprises data concerning medication taken by the patient (and especially still in the patient's body), especially Glucophage or anti-inflammatory drugs or drugs connected with the previously mentioned data (e.g. previous treatments or allergic dispositions).

Suitable data interfaces are known to the skilled person, e.g. RIS (Radiology Information System) or PACS (Picture Archiving and Communication System). In the following the term "dataset" is used for a set comprising patient demographic data, data about previous treatments and data about medication of the patient, wherein this data is used for further determinations. The dataset may also comprise data selected from the group allergic dispositions (of the patient), recent lab results (of examinations of the patient), previous exams and/or diagnosis (of the patient), that may also be used for further determinations.

The risk indicator unit uses the dataset to determine the risk-probability based on the data comprised by the dataset. It preferably determines at least the risk-probability for contrast allergic risks and/or for overdose risks. This could be achieved by a mathematical function weighting scores of the different data types of the dataset (e.g. by a function of the parameters) or by a machine learning model (a "risk prediction model" as described in the following) that had been trained on datasets with respective types of data and labels (as ground truth) whether an incident (especially concerning overdose and/or the occurrence of allergic reaction on a contrast agent).

Suitable output units are known to the skilled person and may e.g. be displays or speakers. The output information may be a risk-score, graphical items indicating a risk (e.g. a red warning sign) or acoustic information e.g. a warning sound. However, the output may also be control data for automatically controlling a CT-system, e.g. reducing the dose if a risk of overdose has been determined or reducing the amount of contrast agent if a risk for an allergic reaction was indicated.

The invention further relates to a method for risk determination for a CT-examination of a patient, comprising the following steps:
- receiving a dataset of a patient, the dataset comprising patient demographic data, data about previous treatments and data about medication of the patient (see above),
- evaluating a risk-probability for the CT-examination of the patient based on the dataset of the patient, in particular based on the patient demographic data, the data about the previous treatments and the data about the medication of the patient, with a risk indicator unit of a risk indicator device according to the invention,
- outputting information and/or control data based on the evaluated risk-probability.

The method for risk determination for a CT-examination of a patient may be, for example, a computer-implemented method.

It should be noted that it is known, what examination should be performed and, thus, information about the planned CT examination, such as radiation dose, intensity, energy and duration of radiation, region of the patient to be examined and contrast agent applied, is well known, also. It is clear that this information is also used for evaluating a risk-probability for a CT-examination of the patient. Thus, for evaluating the risk of an overdose, it is clear that also radiation dose of the planned CT examination, especially as well as radiation energy and other known parameters, is used for evaluation. For evaluating risk of allergic reactions, information about the type and dose of contrast agent that should be applied for the examination is used for evaluation. With the output (of control data) a CT-system may be controlled by inputting the output information into the control device of the CT-system.

Thus, risk factors can be shown directly to a CT operator and/or can be used to control a CT-system.

A risk prediction model according to the invention is designed for risk-prediction, especially designed for a risk indicator device according to the invention or designed to perform a method according to the invention. The risk prediction model is a machine learning model that is trained on a multiplicity of training datasets. Each training dataset comprising at least:
- Patient demographic data, data about previous treatments and data about medication of a patient, and especially also data about allergic dispositions and/or recent lab results and/or previous exam and diagnosis of this patient, and
- A ground truth, indicating at the respective training dataset the occurrence of an incident, especially concerning overdose and/or the occurrence of allergic reaction on a contrast agent.

The risk prediction model has preferably the architecture of a decision tree. The risk prediction model could also be a multivariate analysis.

A patient scheduler according to the invention comprises a risk indicator device according to the invention and is preferably designed to perform a method according to the invention. A "patient scheduler" in the sense of the invention is a software module for scheduling patients, i.e. planning CT-exams for a number of patients. The risk indication device is preferably implemented in form of a risk indication function in the patient scheduler, e.g. integrated in the program code with its functionality. It could also be connected to the patient scheduler via a data interface connected to an external risk indication device (e.g. in a cloud).

that determines whether there is a risk or not (or determines the probability for a risk). Or could the integration be performed in another way, e.g. by contacting an external device hosting the risk prediction model?)
Yes, the function should be coded in the CT console with access to external data sources

A control device according to the invention for controlling a CT-system comprises a risk indicator device according to the invention. Alternatively or additionally it is designed to perform the method according to the invention. The control device may comprise additional units or devices for controlling components of a CT-system.

A CT-system (computing tomography system) according to the invention comprises a risk indicator device according to the invention and especially a control device according to the invention.

Furthermore, we believe that not all data types are used for every sort of risk, since e.g. allergic dispositions are probably not used to evaluate radiation-connected risks. Thus, we believe that a device only used for one special risk does not use all of the listed data types.

Some units or modules of the risk indicator device or the control device mentioned above can be completely or partially realized as software modules running on a processor of a computer or a control device. A realization largely in the form of software modules can have the advantage that applications already installed on a computer can be updated, with relatively little effort, to install and run these units of the present application. The object of the invention is also achieved by a computer program product with a computer program that is directly loadable into the memory of a computer of a medical imaging system, and which comprises program units to perform the steps of the inventive method when the program is executed by the computer. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently installed carrier can serve to transport and/or to store the executable parts of the computer program product so that these can be read from a processor unit of a control device or a system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

According to a preferred risk indicator device, its risk indicator unit comprises a risk prediction model trained for risk prediction (see above). This risk prediction model allows a very accurate and easy risk prediction. However, its training demands some effort.

According to a preferred risk indicator device, the risk indicator unit is designed to evaluate the risk-probability of an overdose and/or of contrast allergic reactions of a patient in the course of a following CT-examination. It is preferred that the risk indicator unit is designed to evaluate the risk-probability of contrast allergic reactions by using additional information of the dataset concerning allergic dispositions and/or recent lab results and/or previous exam and diagnosis of the patient.

A preferred risk indicator device is designed to control a CT-system with the control data. This could be easily achieved by including the risk indicator device in above-described control device or connecting the risk indicator device with such control device. The control data of the output of the risk indicator device should be designed such that they are able to control a CT-system. However, a control device may also act as interpreter for a CT-system for interpreting the control data of the risk indicator device. This has the advantage that the risk-probability is not only indicated but may have a direct influence on the control of a CT-system. Preferably, an examination could only be performed when there is no risk, or the risk-probability lies below a certain threshold. In the case of a risk or a risk-probability above a certain threshold, the system is preferably designed to blocking examination and/or automatically adjusting scan parameters and/or triggering an auditing mechanism or a notification mechanism. This may be achieved by directly controlling the CT-system or by communication with the control device of a CT-system. For example, a CARE-Alert may be outputted what has a direct influence on the control of a CT-system.

A preferred risk indicator device comprises a data interface designed to receive medical data types of different structures, especially a FHIR-interface (FHIR: "Fast Healthcare Interoperability Resources"), for accessing data from different sources. Preferred data types are data from electronic medical records (EMR), Cardiovascular Information Systems (CVIS), Radiology Information Systems (RIS) or Laboratory information systems (LIS). Regardless of the data sources, be it in EMR, RIS or LIS, one can use a standard FHIR interface query and implementation guide to fetch the data to the risk indicator device.

The result could be fed to further systems, e.g. "myExam Companion" and other scan automation mechanisms.

A preferred risk indicator device is designed to output control data for a CT-system. This control data is preferably designed to automatically adjust contrast injection parameters and/or scan parameters depending on the evaluated risk-probability. Thus, dose of radiation or of contrast agent will be adjusted automatically to keep the patient safe from harm automatically.

According to a preferred risk indicator device, the risk indicator unit is designed to evaluate risk-probability by using multivariate analysis.

According to a preferred method, the risk-probability (preferably at least for a risk of overdose) is determined based on
- patient demographic data, especially gender, age, weight, race, pregnancy
- data about previous treatments, especially central venous catheter, kidney surgery, chemotherapy, dialysis, and
- data about medication, especially Glucophage or anti-inflammatory drugs. It should be noted that this concerns the patient that is being examined by the CT-examination.

Preferably, the method is designed to determine the risk of contrast allergic reactions of a patient (alone or along with other risks), wherein the risk-probability is preferably also determined based on
- allergic dispositions, especially asthma, iodine, beta blocker and/or
- recent lab results, especially creatinine level, and/or
- previous exam and diagnosis, especially hypertension, heart failure, myeloma, diabetes,
of the respective patient.

For example, a risk-probability of contrast-induced nephropathy is evaluated by using multivariate analysis. This is achieved by looking at the basal Serum creatinine, appearance of a shock, gender (e.g. female), multivessel PCI, and diabetes mellitus as input parameters.

According to a preferred method, the risk indicator device comprises a risk prediction model. This risk prediction model is trained online by providing a dataset of a patient actually examined in a CT-system and data about incidents during the examination. It is especially preferred that a risk-probability for this patient is determined before the examination and compared with possible incidents during examination after the examination. Thus, online-learning is possible.

The method may also include elements of "cloud computing". In the technical field of "cloud computing", an IT infrastructure is provided over a data-network, e.g. a storage space or processing power and/or application software. The communication between the user and the "cloud" is achieved by means of data interfaces and/or data transmission protocols.

In the context of "cloud computing", in a preferred embodiment of the method according to the invention, provision of data via a data channel (for example a data-network) to a "cloud" takes place. This "cloud" includes a (remote) computing system, e.g. a computer cluster that typically does not include the user's local machine. This cloud can be made available in particular by the medical facility, which also provides the medical imaging systems. In particular, the image acquisition data is sent to a (remote) computer system (the "cloud") via a RIS (Radiology Information System) or a PACS (Picture Archiving and Communication System).

Within the scope of a preferred embodiment of the risk indicator device according to the invention, this device, at least the risk prediction module, are present on the "cloud" side. A preferred system further comprises, a local computing unit connected to the system via a data channel (e.g. a data-network, particularly configured as RIS or PACS). The local computing unit includes at least one data receiving interface to receive data. Moreover, it is preferred if the local computer additionally has a transmission interface in order to send data to the system.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a simplified CT system with a system according to an embodiment of the invention.
Figure 2 shows a block diagram of the process flow of a preferred method according to the invention.
Figure 3 shows a simplified example for the usage of a dataset.
Figure 4 shows a usage of different data types.
Figure 5 shows a possible output of the risk indicator device in a patient scheduler.

In the diagrams, like numbers refer to like objects throughout. Objects in the diagrams are not necessarily drawn to scale.

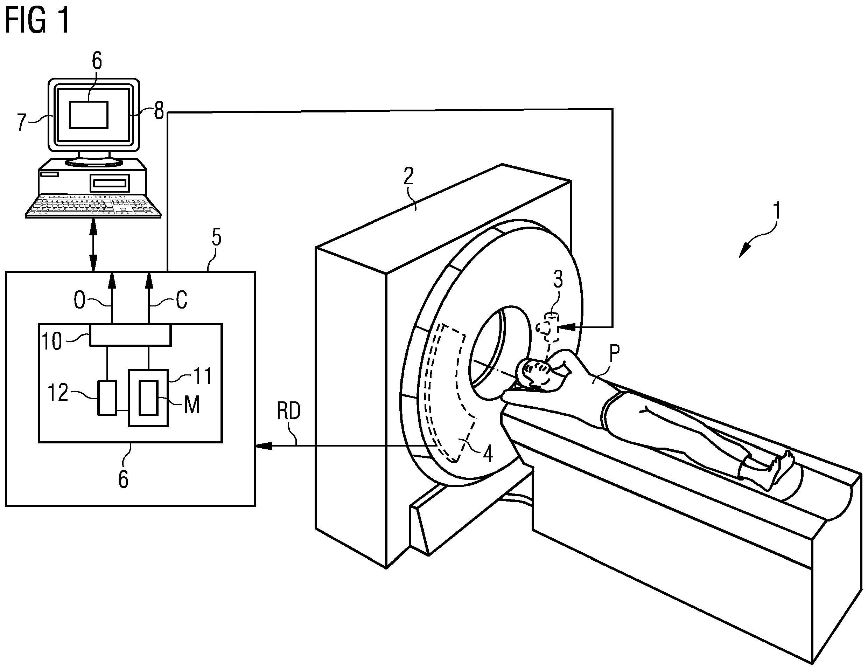

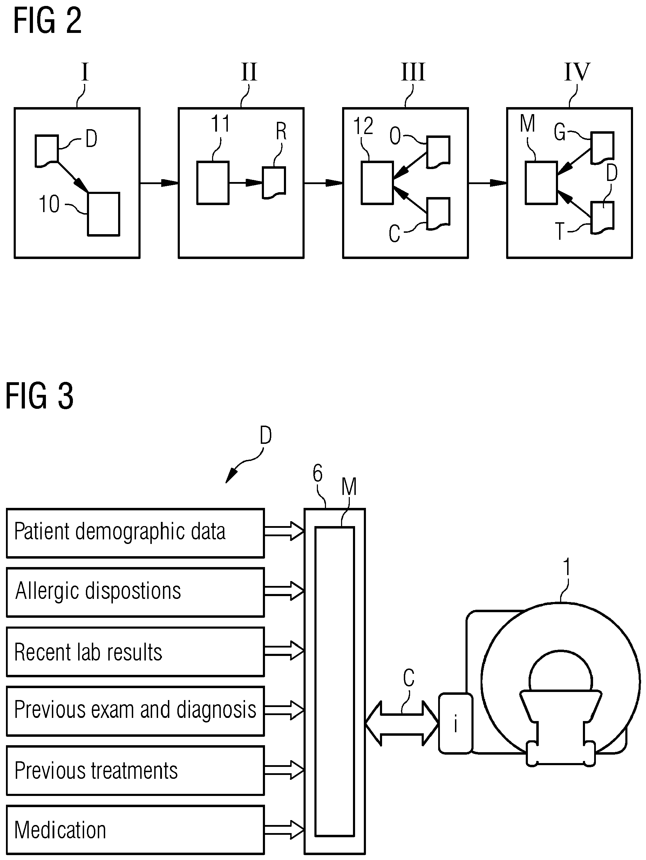

Figure 1 shows a simplified computer tomography system 1 with a control device 5 comprising a risk indicator device 6 for risk determination for a CT-examination, designed to perform the method according to the invention (see figure 2). The computer tomography system 1 has in the usual way a scanner 2 with a gantry, in which an x-ray source 3 with a detector 4 rotates around a patient and records raw data RD that is later reconstructed to images by the control device 5. A patient P lying on a patient bed ready for examination.

In this figure, only those components are shown that are essential for explaining the invention. In principle, such imaging systems and associated control devices are known to the person skilled in the art and therefore do not need to be explained in detail.

A user can interact with the computer tomography system 1 by using terminal 7 that is able to communicate with the control device 5. This terminal 7 can also be used to examine results of the risk indicator device 6 according to the invention or to provide data for the risk indicator device 6.

Although the risk indicator device 6 is shown here in the control device of the CT-system, it could also be present in the terminal 7, e.g. in the patient scheduler 8 as indicated.

The system 6 comprises the following components:
A data interface 10 designed for receiving a dataset D (see figure 2) of the patient P to be examined. The data interface 10 shown here is also used for communicating with the CT-system 1 and sending output information O and control data C from the output unit 12 of the risk indicator device 6 to the CT-system 1.

Preferably, the data interface 10 is designed to receive medical data types of different structures. It is preferably a FHIR-interface, for accessing data from different sources. Preferred data types are data from electronic medical records, Cardiovascular Information Systems, Radiology Information Systems or Laboratory information systems.

A risk indicator unit 11 designed to evaluate a risk-probability R for the CT-examination. The risk indicator unit 11 here comprises a risk prediction model M trained for risk prediction. Preferably, the risk-probability R of an overdose and/or of contrast allergic reactions of the patient P in the course of the following CT-examination are evaluated.

An output unit 12 that is designed to output output information O and/or control data C based on the evaluated risk-probability R. When outputting control data C, the control data C is preferably designed to automatically adjust contrast injection parameters and/or scan parameters, depending on the evaluated risk-probability R.

The components of the system preferably appear to be software modules.

Figure 2 shows a block diagram of the process flow of a preferred method for risk determination for a CT-examination of a patient.

In step I, a dataset D of a patient P is received by the data interface 10 of the risk indicator device 6 (see figure 1). The dataset D comprises patient demographic data, data about previous treatments and data about medication of the patient. In step II, a risk-probability R for a CT-examination is evaluated based on the patient demographic data, data about previous treatments and data about the medication of the patient. This is achieved with the risk indicator unit 11 of the risk indicator device 6.

In step III output information O and control data C are outputted based on the evaluated risk-probability R.

In step IV, a risk prediction model M of the risk indicator device 6 (see figure 1) is trained online by providing a dataset D of the patient P examined actually examined in the CT-system 1. This dataset D is here used as training dataset T. In addition, a ground truth G about incidents during the CT-examination is added to train the risk prediction model M.

Figure 3 shows a simplified example for the usage of a dataset D by a risk prediction model M of a risk indicator device 6. Patient demographic data, data about allergic dispositions, recent lab results and previous CT-examination and diagnosis as well as data about previous treatments and medication are inputted into the risk prediction model M. A risk-probability R is then evaluated by the risk prediction model M, e.g. by a method as shown in figure 2, and depending on the evaluated risk-probability R, control data C is sent to a CT-system 1.

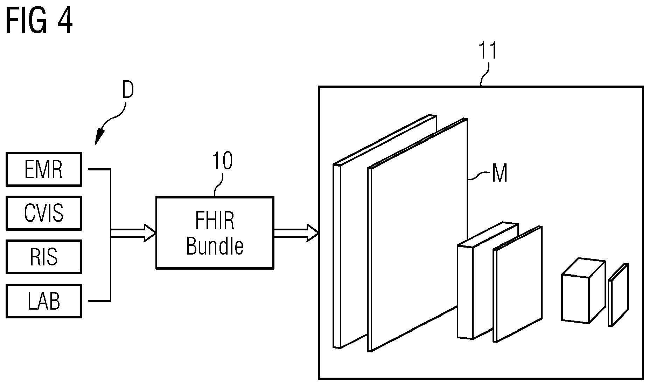

Figure 4 shows a usage of different data types. The risk indicator device 6 here comprises a special data interface 10 (FHIR-interface here designated as "FHIR bundle") that is designed to receive medical data types of different structures. The inputted dataset D here comprises data from electronic medical records "EMR", Cardiovascular Information Systems "CVIS", radiology information systems "RIS" and lab data from laboratory information systems "LAB". This data is used by a risk prediction model M in a risk indicator unit 11.

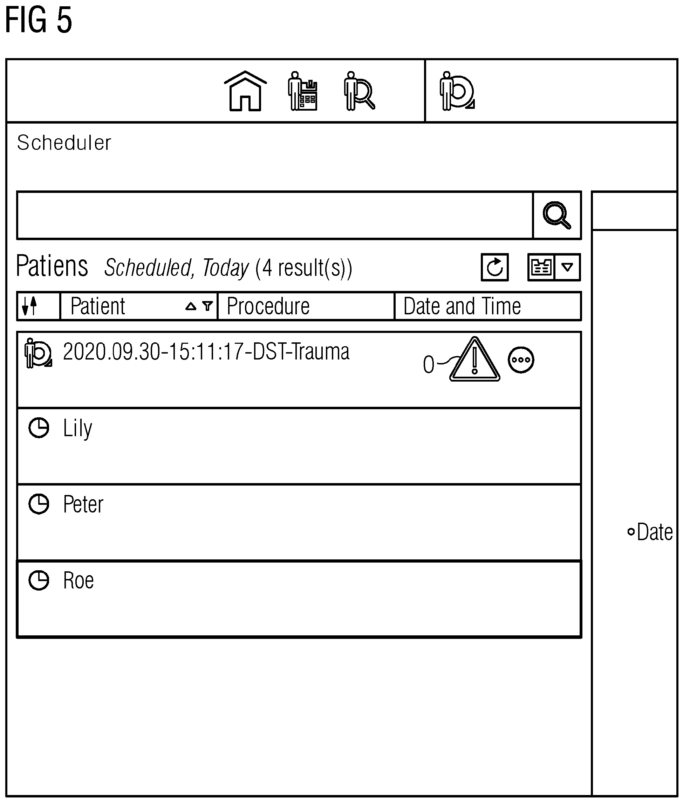

Figure 5 shows a possible output of the risk indicator device 6 in a patient scheduler 8 (see figure 1). Here a possible output of the patient scheduler 8 is shown with a warning sign as output information O indicating a certain risk for a patient P for the next CT examination.
Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The mention of a "unit" or a "device" does not preclude the use of more than one unit or device.

## Claims

1. A risk indicator device (6) designed for risk determination for a CT-examination, comprising:
- a data interface (10) designed for receiving a dataset (D) of a patient (P), the dataset (D) comprising patient demographic data, data about previous treatments and data about medication of the patient (P),
- a risk indicator unit (11) designed to evaluate a risk-probability (R) for the CT-examination based on the patient demographic data, the data about the previous treatments and the data about the medication of the patient (P),
- an output unit (12) designed to output output information (0) and/or control data (C) based on the evaluated risk-probability (R).

2. The risk indicator device according to claim 1, wherein the risk indicator unit (11) comprises a risk prediction model (M) trained for risk prediction.

3. The risk indicator device according to any of the preceding claims, wherein the risk indicator unit (11) is designed to evaluate the risk-probability (R) of an overdose and/or of contrast allergic reactions of a patient (P) in the course of a following CT-examination,
preferably wherein the risk indicator unit (11) is designed to evaluate the risk-probability (R) of contrast allergic reactions by using additional information of the dataset (D) concerning allergic dispositions and/or recent lab results and/or previous CT-examination and diagnosis of the patient (P) .

4. The risk indicator device according to any of the preceding claims, comprising
a data interface (10) designed to receive medical data types of different structures, especially a FHIR-interface, for accessing data from different sources, wherein preferred data types are data from electronic medical records, cardiovascular information systems, radiology information systems or laboratory information systems.

5. The risk indicator device according to any of the preceding claims, designed to output control data (C) for a CT-system, wherein the control data (C) is preferably designed to automatically adjust contrast injection parameters and/or scan parameters, depending on the evaluated risk-probability (R).

6. The risk indicator device according to any of the preceding claims, wherein the risk indicator unit (11) is designed to evaluate risk-probability (R) by using multivariate analysis.

7. A method for risk determination for a CT-examination of a patient, comprising the steps:
- receiving a dataset (D) of a patient (P), the dataset (D) comprising patient demographic data, data about previous treatments and data about medication of the patient,
- evaluating a risk-probability (R) for the CT-examination of the patient (P), based on the patient demographic data, the data about the previous treatments and the data about the medication of the patient, with a risk indicator unit (11) of a risk indicator device (6) according to any of the preceding claims,
- outputting output information (0) and/or control data (C) based on the evaluated risk-probability (R).

8. The method according to claim 7, wherein the risk probability (R) is determined based on
- patient demographic data, especially gender, age, weight, race, pregnancy
- data about previous treatments, especially central venous catheter, kidney surgery, chemotherapy, dialysis, and
- data about medication, especially Glucophage or anti-inflammatory drugs,
concerning the respective patient being examined by the CT-examination,
wherein the method is preferably designed to determine the risk of overdose.

9. The method according to claim 7 or 8, wherein the method is designed to determine the risk-probability (R) of contrast allergic reactions of a patient (P), wherein the risk-probability (R) is preferably determined also based on
- allergic dispositions, especially asthma, iodine, beta blocker and/or
- recent lab results, especially creatinine level, and/or
- previous CT-examination and diagnosis, especially hypertension, heart failure, myeloma, diabetes,
of the respective patient (P).

10. The method according to any of claims 7 to 9, wherein the risk indicator device (6) comprises a risk prediction model (M) that is trained online by providing a dataset (D) of a patient (P) actually examined in a CT-system (1) and data about incidents during the CT-examination, especially wherein a risk-probability (R) for this patient (P) is determined before the CT-examination and compared with possible incidents during CT-examination after the CT-examination.

11. A risk prediction model (M) designed for risk-prediction, preferably designed for a risk indicator device (6) according to any of claims 1 to 6 and especially to perform risk prediction in a method according to any of claims 7 to 10, wherein the risk prediction model (M) is a machine-learning model trained on a multiplicity of training datasets (T), each training dataset (T) comprising at least
- patient demographic data, data about previous treatments and data about medication of a patient (P), and especially also data about allergic dispositions and/or recent lab results and/or previous CT-examination and diagnosis of this patient (P), and
- a ground truth, indicating at the respective training dataset (T) the occurrence of an incident, especially concerning overdose and/or the occurrence of allergic reaction on a contrast agent,
wherein the risk prediction model (M) preferably has the architecture of a decision tree.

12. A patient scheduler (8) comprising a risk indicator device (6) according to any of claims 1 to 6 and preferably designed to perform a method according to any of claims 7 to 10.

13. CT-system (1) comprising a risk indicator device (6) according to any of claims 1 to 6 and preferably designed to perform a method according to any of claims 7 to 10.

14. A computer program product comprising a computer program that is directly loadable into a patient scheduler (8) or a control device (13) for a magnetic resonance imaging system (1), which comprises program elements for performing steps of the method according to any of claims 7 to 10 when the computer program is executed by the patient scheduler (8) or the control device (13).

15. A computer-readable medium on which is stored program elements that can be read and executed by a computer unit in order to perform steps of the method according to any of claims 7 to 10, when the program elements are executed by the computer unit.
